Europäisches Patentamt

European Patent Office

Office européen des brevets

(19)

(11) Veröffentlichungsnummer: **0 377 401**

**A1**

(12) **EUROPÄISCHE PATENTANMELDUNG**

(21) Anmeldenummer: 89730224.6

(22) Anmeldetag: 20.12.89

(51) Int. Cl.⁵ **A61B 17/58**

(30) Priorität: 04.01.89 DE 8900121 U

(43) Veröffentlichungstag der Anmeldung:
**11.07.90 Patentblatt 90/28**

(84) Benannte Vertragsstaaten:
**AT BE CH DE ES FR GB IT LI NL SE**

(71) Anmelder: **MECRON MEDIZINISCHE PRODUKTE GMBH**
**Nunsdorfer Ring 23-29**
**D-1000 Berlin 48(DE)**

(72) Erfinder: **Anapliotis, Emmanuel**
**Kiebitzweg 5**
**D-1000 Berlin 33(DE)**
Erfinder: **Regener, Gernot**
**Bocksfeldstrasse 1e**
**D-1000 Berlin 20(DE)**
Erfinder: **Ahrens, Uwe, Dipl.-Ing.**
**Nürnberger Strasse 46**
**D-1000 Berlin 30(DE)**
Erfinder: **Jehle, Hermann, Dr.**
**Kreiskrankenhaus Alte Waibstädter Strasse 2**
**D-6920 Sinsheim(DE)**

(74) Vertreter: **Christiansen, Henning, Dipl.-Ing.**
**Patentanwalt Paceliiallee 43/45**
**D-1000 Berlin 33(DE)**

(54) **Kompressionsverschraubung.**

(57) Kompressionsverschraubung für Schenkelhalsbrüche, bestehend aus einer am Femurschaft zu verschraubenden Hülsenlasche (5) einer hohlzylindrischen Hülse (1) und einer darin geführten Knochenschraube (4), die an ihrer Kopfseite mit einem Innengewinde (9) versehen ist, in welchem eine mit einem entsprechenden Außengewinde versehene Kompressionsschraube (8) eingreift, wobei sich die Kompressionsschraube (8) über die Unterseite ihres Kopfes oder eine entsprechende, insbesondere konische, Anschlagfläche (10) gegen einen Spannring (7) abstützt, der innerhalb der Hülse (1) verschieblich gelagert ist, mit seiner Außenfläche an der Innenfläche der Hülse (1) gleitet und in axialer Richtung eine konische Fläche aufweist, die der Unterseite des Kopfes der Kompressionsschraube (8) angepaßt ist und deren Konuswinkel so bemessen ist, daß die Haftreibungskraft zwischen der Außenfläche des Spannrings (7) und der Innenfläche der Hülse (1) größer ist als die in Längsrichtung der Schraube (8) wirkende Kompressionskraft.

Fig.1

# Kompressionsverschraubung

Die Erfindung betrifft eine Kompressionsverschraubung der im Oberbegriff des Anspruchs 1 angegebenen Art sowie eine Vorrichtung zur Durchführung des Verfahrens.

Die Osteosynthese bei Schenkelhalsbrüchen löst zunehmend den Knochenersatz durch Endoprothesen ab. Dieser Entwicklung wird auch durch eine Vielzahl neu entwickelter Operationsverfahren und Hilfsmittel Rechnung getragen.

Insbesondere durchgesetzt hat sich das Verschrauben und/oder Vernageln von Knochenfragmenten, wobei es sich gezeigt hat, daß eine Druckverspannung die eine Verzahnung und Verkeilung der Bruchflächen bewirkt, die Frakturheilung und insbesondere die Revaskularisierung des Kopffragmentes in diesem mechanisch so gefährdeten Bereich durch eine bessere Stabilisierung und Ausschaltung störender Scher- und Rotationsbewegungen wesentlich verbessert.

Bekannt ist es, die Hülse einer derartigen Kompressionsverschraubung an ihrer Innenseite stufenförmig auszubilden, so daß sich der Kopf der Kompressionsschraube gegen die ringförmige Stirnfläche der Ausfräsung abstützen kann. Da die Einschraubtiefe der Knochenschraube jedoch in Abhängigkeit von Art und Ort der Fraktur sehr unterschiedlich sein kann, muß die Kompressionsschraube bei fester Abstützungsfläche in ihrer Länge den jeweiligen Bedingungen angepaßt sein.

Nachteilig dabei ist, daß im Falle einer nicht ausreichenden oder zu großen Länge der gewählten Schraube, diese zunächst wieder ausgedreht werden muß, um sie durch eine andere Schraube mit abweichender Länge zu ersetzen. Hierdurch wird die Belastung des Patienten unnötigerweise erhöht.

Der Erfindung liegt die Aufgabe zugrunde, eine Kompressionsverschraubung der eingangs genannten Gattung anzugeben, die es gestattet, einheitliche Kompressionsschrauben zu verwenden, wobei insbesondere eine sichere Auflage innerhalb der Hülse zu finden ist, die auch großen axialen Kräften standhält.

Diese Aufgabe wird mit den kennzeichnenden Merkmalen des Anspruchs 1 gelöst.

Der Erfindung liegt die Erkenntnis zugrunde, daß indem innerhalb der Hülse ein Spannring an beliebiger Stelle eingesetzt werden kann, der sich bei Anzug der Kompressionsschraube aufspreizt und sich so stark an die Innenwandung der Hülse anpreßt, daß die Haftreibung ein Gleiten des Spannringes verhindert, eine Längenauswahl oder -anpassung der Schraube selbst nicht mehr erforderlich ist. Die Arretierung wird durch eine axiale konische Ausdrehung des Spannringes erreicht, in der die Unterseite des Kopfes der Kompressionsschraube genau eingreift. Die aufspreizende Wirkung des Spannringes kann entsprechend einer vorteilhaften Weiterbildung der Erfindung noch verstärkt werden durch Schlitzung des Spannringes in Längsrichtung. Der Spannring kann bereits beim Einsetzen in die Hülle durch seine radialen federnden Eigenschaften in die Hülse eingeklemmt und gegen unbeabsichtigtes Verschieben gesichert werden.

Insbesondere vorteilhaft ist auch, daß die erfindungsgemäße Konstruktion die Kompressionsverschraubung nicht vergrößert. Sie ist kostengünstig und ohne wesentlichen Mehraufwand herzustellen, insbesondere, weil sich der Materialaufwand wegen der gegebenenfalls kürzeren Schraube noch verringert. Der Kopf der Kompressionsschraube liegt innerhalb der Hülse und hat einen dementsprechend geringen Durchmesser.

Um den Spannring beim Einschrauben der Kompressionsschraube gegen Verdrehen zu sichern, ist vorzugsweise der Spannring mit einer äußeren Längsnut versehen. Dazu ist entsprechend innerhalb der Hülse in Längsrichtung ein entsprechender Führungssteg vorgesehen, die der Schlitz des Spannringes umgreift. Umgekehrt kann aber auch die Hülse mit einer Längsnut versehen sein, in welcher der Spannring mittels eines Stiftes oder Vorsprungs geführt wird.

Um Gewebeverträglichkeit sicherzustellen, erweist es sich ferner als vorteilhaft, alle Bestandteile des Implantates sowie die mit Körpergewebe in Berührung kommenden Instrumente aus identischem Material, vorzugsweise aus dem körperverträglichen Werkstoff Stahl X 5 CrNiMo 1810, herzustellen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 einen Längsschnitt durch die erfindungsgemäße Kompressionsverschraubung mit andeutungsweiser Darstellung einer Schenkelhalsfraktur;

Figur 2 eine perspektivische Darstellung des Spannringes.

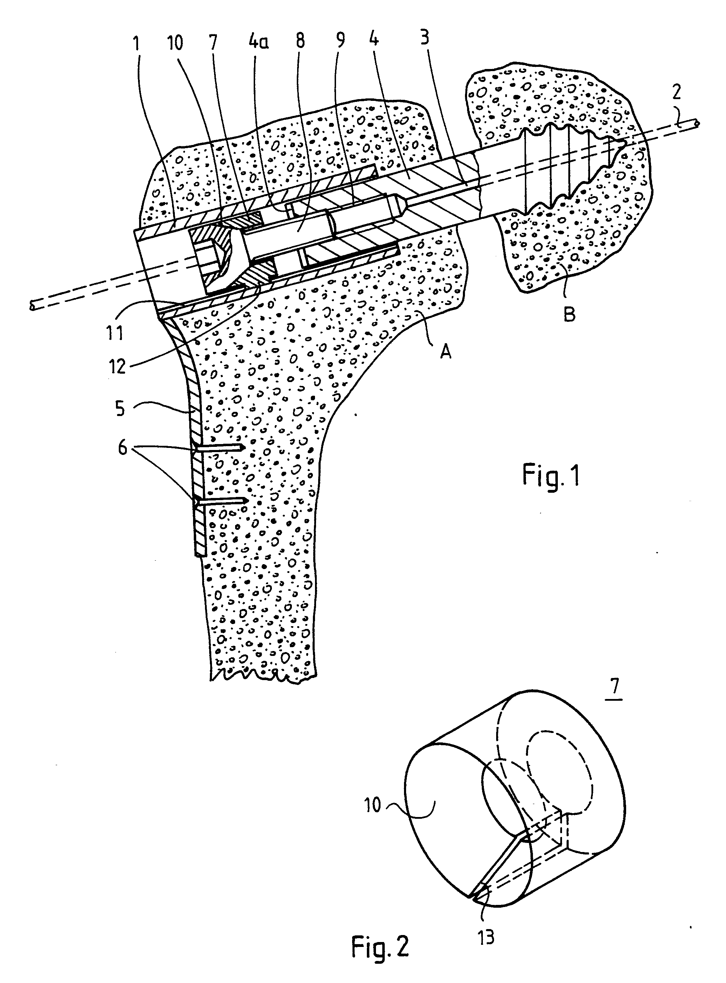

Bei dem in Figur 1 in der Anwendung im Schenkelhalsbereich A, B dargestellten Ausführungsbeispiel unter Wiedergabe einer erfindungsgemäßen Kompressionsverschraubung soll zum Verständnis zunächst das Operationsverfahren beschrieben werden:

Bei der operativen Behandlung von Schenkelhalsfrakturen mittels Osteosynthese wird zunächst eine Hülse 1 in den mit entsprechendem Durchmesser aufgebohrten Femur eingesetzt, durch die dann ein Kirschnerdraht 2 als Führungsspieß eingetrieben wird, um das nachfolgende Einschrauben der mit einer Längsbohrung 3 für den Kirschnerdraht 2 versehenen Knochenschraube 4 zu erleichtern.

Die Schraube 4 wird mittels einer an ihrem rückwärtigen Ende befindlichen Nut 4a, in die zu diesem Zweck ein Schraubendreher eingreifen kann, durch den Frakturquerschnitt hindurch in den Kopfbereich B eingedreht. Anschließend wird der Kirschnerdraht 2 entfernt. Die Hülsenlasche 5 wird formfest an der Hülse angesetzt und durch leichtes Klopfen der Form des Flanierschaftes angepaßt, an welchen sie nach entsprechendem Vorbohren mittels Corticalisschrauben 6 angeschraubt wird.

Abhängig von der erreichten Einschraubtiefe der Knochenschraube 4 wird der Spannring 7 in der Hülse 1 unter Berücksichtigung der Länge einer Schraube 8, die in ein Innengewinde 9 der Knochenschraube 4 mittels einem Maschinengewinde eindrehbar ist, positioniert. Dieser ist zunächst innerhalb der Hülse frei verschiebbar. Anschließend erfolgt das Einschrauben der Kompressionsschraube 8 in das dafür vorgesehene Innengewinde 9 an dem in die Hülse 1 hineinragenden Ende der Knochenschraube 4. Um das Aufspreizen des Spannringes 7 beim Einschrauben der Kompressionsschraube 8 zu erreichen, weisen der Spannring 7 und die Unterseite der Kompressionsschraube 8 an ihrer Berührungsfläche 10 eine angepaßte konische Form auf.

Die mit dem Aufspreizen der Hülse durch die Keilwirkung des Konus entstehende Haftreibungskraft ist größer als die in Längsrichtung der Schraube wirkende Kompressionskraft. Damit ist die Hülse selbsthemmend, so daß die Kompressionskraft die Knochenbereiche A und B einander in der gewünschten Weise annähert.

Die Hülse 1 ist an ihrer Innenseite mit einer Längsnut 11 versehen, in die ein Führungsstift 12 des Spannringes 7 eingreift, wodurch das Verdrehen des Spannringes 7 beim Festziehen der Kompressionsschraube 8 verhindert wird. Ein entsprechender Effekt läßt sich erzielen, wenn, wie in Figur 2 dargestellt, der Spannring 7 einen Längsschlitz 13 aufweist und dieser in einer Führungsschiene, die in die Hülse 1 eingesetzt wird, verschiebbar ist. Der Längsschlitz 13 verbessert dabei zusätzlich die Aufspreiz- und Klemmeigenschaften des Spannringes 7. Nach dem Verheilen des Bruches wird das gesamte Implantat entfernt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Ansprüche

1. Kompressionsverschraubung für Schenkelhalsbrüche, bestehend aus einer am Femurschaft zu verschraubenden Hülsenlasche einer hohlzylindrischen Hülse und einer darin geführten Knochenschraube, die an ihrer Kopfseite mit einem Innengewinde versehen ist, in welchem eine mit einem entsprechenden Außengewinde versehene Kompressionsschraube eingreift, **dadurch gekennzeichnet,** daß sich die Kompressionsschraube über die Unterseite ihres Kopfes oder eine entsprechende, insbesondere konische, Anschlagfläche gegen einen Spannring abstützt, der innerhalb der Hülse verschieblich gelagert ist, mit seiner Außenfläche an der Innenfläche der Hülse gleitet und in axialer Richtung eine konische Fläche aufweist, die der Unterseite des Kopfes der Kompressionsschraube angepaßt ist und deren Konuswinkel so bemessen ist, daß die Haftreibungskraft zwischen der Außenfläche des Spannrings und der Innenfläche der Hülse größer ist als die in Längsrichtung der Schraube wirkende Kompressionskraft.

2. Kompressionsverschraubung nach einem der vorhergehenden Ansprüche, **dadurch gekennzeichnet,** daß der Spannring in Längsrichtung geschlitzt ausgebildet ist.

3. Kompressionsverschraubung nach einem der vorhergehenden Ansprüche, **dadurch gekennzeichnet,** daß eine längs gerichtete Führung zur Sicherung des Spannrings gegen Verdrehen innerhalb der Hülse vorgesehen ist.

4. Kompressionsverschraubung nach Anspruch 3, **dadurch gekennzeichnet,** daß die Führung aus einer Längsnut des Spannrings besteht, die in einen längsgerichteten, an der Innenseite der Hülse vorgesehenen Steg eingreift.

5. Kompressionsverschraubung nach Anspruch 3, **dadurch gekennzeichnet,** daß der Spannring mit einem stiftförmigen, radial nach außen gerichteten Stift versehen ist, der in die an der Innenseite der Hülse vorgesehene Längsnut eingreift.

6. Kompressionsverschraubung nach einem der vorangehenden Ansprüche, **dadurch gekennzeichnet,** daß alle Bestandteile des Implantats und alle mit Körpergewebe in Berührung kommenden Instrumente aus dem gleichen Material, vorzugsweise aus dem X 5 CrNiMo 1810, gefertigt sind.

ME 36.18-EU

Fig.1

Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 085 493 (RICHARDS MANUFACTURING CO.)<br>* Zusammenfassung *<br>--- | 1 | A 61 B 17/58 |
| A | US-A-4 432 358 (I.E. FIXEL)<br>* Zusammenfassung; Abbildung 2 *<br>--- | 1 | |
| A | US-A-3 107 666 (E. CECERE et al.)<br>* Spalte 3, Zeilen 9-27; Abbildung 2 *<br>--- | 1 | |
| A | EP-A-0 023 228 (M. STÜRMER)<br>* Seite 3, Zeilen 15-22; Abbildung 2 *<br>--- | 1 | |
| A | US-A-3 029 811 (C.E. YOST)<br>* Spalte 2, Zeilen 17-19; Abbildung *<br>----- | 3,5 | |

**RECHERCHIERTE SACHGEBIETE (Int. Cl.5)**

A 61 B

Der vorliegende Recherchenbericht wurde für alle Patentansprüche erstellt

| Recherchenort | Abschlußdatum der Recherche | Prüfer |
|---|---|---|
| DEN HAAG | 19-03-1990 | WOLF C.H.S. |